# EUROPEAN PATENT APPLICATION

(11) **EP 4 420 737 A1**
(43) Date of publication of application: **28.08.2024**
(21) Application number: 23158128.1
(22) Date of filing: 23.02.2023
(51) Int. Cl.: A61Q 11/00, A61K 8/27, A61K 8/44

(54) **ORAL CARE COMPOSITION**

(71) Applicant: Unilever IP Holdings B.V., 3013 AL Rotterdam (NL)
(72) Inventor: GUAGLIARDO, Roberto, 6708 WH Wageningen (NL); SAVOCA, Michela, 6708 WH Wageningen (NL)
(74) Representative: Tansley, Sally Elizabeth

(57) **Abstract**

A toothpaste composition comprising:
i) a zinc salt and
ii) disodium cocoyl glutamate and sodium olivoyl glutamate.

## Description

### Field of the Invention

The present invention relates to a toothpaste composition with good foaming properties.

### Background of the Invention

Zinc salts have been used in some oral care compounds for a number of years.

US 5 470 561 discloses an anti-plaque mouthwash comprising a zinc salt and triclosan. The composition may also comprise glycine and has a pH of between 4 and 8, preferably between 5 and 7, the preferred pH being 6.

Toothpaste compositions comprising zinc are disclosed in WO0612977. The toothpaste comprises water, a zinc salt, a chelating agent for the zinc.

US 5 632 972 discloses a method for minimising damage to gingival and periodontal tissue by delivering a first component comprising zinc and a second component comprising a bicarbonate.

GB 2 052 978 discloses oral care compositions comprising zinc-glycine combinations.

Zinc salts have a tendency to make a formulation astringent, the present invention describes compositions that mitigate this astringency.

### Description of the Invention

The invention relates to a toothpaste composition comprising:
i) a zinc salt and
ii) disodium cocoyl glutamate and sodium olivoyl glutamate.

### Detailed Description of the Invention

Except in the examples, or where otherwise explicitly indicated, all numbers in this description indicating amounts of material or conditions of reaction, physical properties of materials and/or use may optionally be understood as modified by the word "about".

All amounts are by weight of the composition, unless otherwise specified.

It should be noted that in specifying any ranges of values, any upper value can be associated with any particular lower value.

Where a feature is disclosed with respect to a particular aspect of the invention (for example a composition of the invention), such disclosure is also to be considered to apply to any other aspect of the invention (for example a method of the invention) mutatis mutandis.

Any ingredients mentioned in this application that are natural or naturally derived have been sourced from Europe.

The term "oral care composition" refers to a composition that is delivered to the oral surfaces. The composition may be a product which, during the normal course of usage, is not for the purpose of systemic administration or intentionally swallowed but is rather retained in the oral cavity for a time sufficient to contact substantially all of the dental surfaces and/or oral tissues for the purposes of oral activity.

Sodium olivoyl glutamate /disodium cocoyl glutamate combination is available commercially as olive oil glutamate (olivoil glutamate ex Kalichem).

A method of preparing sodium olivoyl glutamate disodium cocoyl glutamatei is described in US 6 512 093.

The composition of the invention is used to preferably used to clean the surfaces of the oral cavity.

Accordingly, preferred product forms for compositions of the invention are those which are suitable for brushing and/or rinsing the surfaces of the oral cavity.

The composition of the invention is in the form of a toothpaste. The term "dentifrice" denotes an oral composition which is used to clean the surfaces of the oral cavity. Such a composition is not intentionally swallowed for purposes of systemic administration of therapeutic agents, but is applied to the oral cavity, used to treat the oral cavity and then expectorated. Typically such a composition is used in conjunction with a cleaning implement such as a toothbrush, usually by applying it to the bristles of the toothbrush and then brushing the accessible surfaces of the oral cavity.

Preferably the toothpaste is in the form of an extrudable semi-solid such as a cream, paste or gel (or mixture thereof).

Compositions of the invention comprise sodium olivoyl glutamate /disodium cocoyl glutamate. It is preferred if the level of sodium olivoyl glutamate /disodium cocoyl glutamate is from is from 0.05 wt% to 4 wt%, preferably 0.5 to 2.5 wt% of the total composition.

It is preferred if the level of sulphate based surfactant, particularly sodium lauryl sulphate is less than the level of sodium olivoyl glutamate /disodium cocoyl glutamate. It is particularly preferred if the level of disodium cocoyl glutamate and/or sodium olivoyl glutamate comprise at least 75 wt% of the total surfactant present.

Compositions according to the invention comprise a water-soluble or sparingly water-soluble zinc salts such as zinc chloride, zinc acetate, zinc gluconate, zinc sulphate, zinc fluoride, zinc citrate, zinc lactate, zinc oxide, zinc monoglycerolate, zinc tartrate, zinc pyrophosphate and zinc maleate; zinc citrate is particularly preferred.

Preferably the level of zinc salt is from 0.1 to 5 w5% of the total composition, more preferably 1 to 3 wt%.

A composition according to the invention (such as a dentifrice/toothpaste) will generally contain further ingredients to enhance performance and/or consumer acceptability, in addition to the ingredients specified above.

Compositions according to the invention, particularly toothpastes, preferably comprise particulate abrasive materials such as silicas, aluminas, calcium carbonates, dicalciumphosphates, calcium pyrophosphates, hydroxyapatites, trimetaphosphates, insoluble hexametaphosphates and so on, including agglomerated particulate abrasive materials, usually in amounts between 3 and 60% by weight of the oral care composition.

In one embodiment of the invention the composition comprises a silica based abrasive. The preferred abrasive silicas used in the present invention is a silica with a low refractive index. It may be used as the sole abrasive silica, or in conjunction with a low level of other abrasive silicas, e.g. those according to EP 236070. The low refractive index silicas, used as abrasives in the present invention are preferably silicas with an apparent refractive index (R.I.) in the range of 1.41 -1.47, preferably 1.435 - 1.445, preferably having a weight mean particle size of between 5 and 15 mm, a BET (nitrogen) surface area of between 10 and 100 m2/g and an oil absorption of about 70 - 150 cm3/100 g, but abrasive silicas with a lower apparent refractive index may also be used. Typical examples of suitable low refractive index abrasive silicas (e.g. having an R.I. of between 1.435 and 1.445) are Tixosil 63 and 73 ex Rhone Poulenc; Sident 10 ex Degussa; Zeodent 113 ex Zeofinn; Zeodent 124 ex Evonik, Sorbosil AC 77 ex PQ Corporation (having an R.I. of approximately 1.440). The amount of these silicas in the composition generally ranges from 5-60% by weight, usually 5-20% by weight.

An alternative suitable abrasive is calcium carbonate. Natural calcium carbonate abrasive is typically a finely ground limestone which may optionally be refined or partially refined to remove impurities. The material preferably has an average particle size of less than 10 microns, e.g., 3-7 microns, e.g., about 5.5 microns. For example, a small particle silica may have an average particle size (D50) of 2.5 -4.5 microns. Because natural calcium carbonate may contain a high proportion of relatively large particles of not carefully controlled, which may unacceptably increase the abrasivity, preferably no more than 0.01 wt%, preferably no more than 0.004 percent by weight of particles would not pass through a 325 mesh. The material has strong crystal structure, and is thus much harder and more abrasive than precipitated calcium carbonate. The tapped density for the natural calcium carbonate is for example between 1 and 1.5 g/cc, e.g., about 1.2 for example about 1.19 g/cc. There are different polymorphs of natural calcium carbonate, e.g., calcite, aragonite and vaterite, calcite being preferred for purposes of this invention.

Precipitated calcium carbonate has a different crystal structure from natural calcium carbonate. It is generally more friable and more porous, thus having lower abrasivity and higher water absorption. For use in the present invention, the particles are small, e.g., having an average particle size of 1-5 microns, and e.g., no more than 0.1 percent, preferably no more than 0.05 percent by weight of particles which would not pass through a 325 mesh. The particles may for example have a D50 of 3-6 microns, for example 3.8-4.9, e.g., about 4.3; a D50 of 1-4 microns, e.g., 2.2-2.6 microns, e.g., about 2.4 microns, and a D10 of 1-2 microns, e.g., 1.2-1.4, e.g., about 1.3 microns. The particles have relatively high water absorption, e.g., at least 25 g/100 g, e.g., 30-70 g/100 g.

In some embodiments, additional calcium-containing abrasives, for example calcium phosphate abrasive, e.g., tricalcium phosphate, hydroxyapatite or dicalcium phosphate dihydrate or calcium pyrophosphate, and/or silica abrasives, sodium metaphosphate, potassium metaphosphate, aluminum silicate, calcined alumina, or other siliceous materials, or combinations thereof are used. The composition, particularly if a toothpaste preferably comprises an inorganic or a natural or synthetic thickener or gelling agent in proportions of about 0.10 to about 15% by weight depending on the material chosen. These proportions of thickeners in the dentifrice compositions of the present invention form an extrudable, shape-retaining product which can be squeezed from a tube onto a toothbrush and will not fall between the bristles of the brush but rather, will substantially maintain its shape thereon. Suitable thickeners or gelling agents useful in the practice of the present invention include inorganic thickening silicas such as amorphous silicas available from Huber Corporation under the trade designation Zeodent 165, Irish moss, iota-carrageenan, gum tragacanth, and polyvinylpyrrolidone.

Further suitable binders and thickeners can be present and include as sodium carboxymethylcellulose, hydroxyethyl cellulose (Natrosol^{®}), xanthan gum, gum arabic etc. as well as synthetic polymers such as polyacrylates and carboxyvinyl polymers such as Carbopol^{®}.

Compositions according to the invention may comprise a polymeric deposition aid. Preferably the composition comprises acid anhydride polymers, particularly preferred are co-polymers of maleic anhydride with methyl vinylether, in which the anhydride moiety may be in a partially or fully hydrolysed or alcoholysed form. Preferred copolymers include Gantrez(R) polymers such as:
Gantrez S-95: molecular weight 216,000; free acid;
Gantrez S-96: molecular weight 700,000; free acid;
Gantrez S-97: molecular weight 1,500,000; free acid; and
Gantrez MS-955: molecular weight 1,060,000; calcium/sodium salt.
Particularly preferred co-polymers of maleic acid and methyl vinylether have a molecular weight of 1,000,000 or greater and an especially preferred material is Gantrez S-97.

Compositions according to the invention may comprise a tooth whitening agent. The whitening agent preferably comprises a green and/or a blue pigment. In the context of the present invention a pigment is generally understood to be a shade/material which is insoluble in the relevant medium, at the relevant temperature. This is in contrast to dyes which are soluble. In the context of this invention, the "relevant medium" is human saliva, the liquid medium in which the composition is used, at the temperature of the oral cavity during brushing of the teeth, i.e. up to 37 Degrees C. As a reasonable approximation, the relevant medium may be considered to be water and the relevant temperature to be 25 Degrees C.

Preferably the blue pigment is Pigment Blue 15, more preferably Pigment Blue 15:1, 15:2, 15:3, 15:4, 15:5 or 15:6, most preferably 15:1. A preferred pigment is blue pigment is Phthalocyanine Blue Pigment, CI No. 74160, blue covarine.

The preferred Green pigment is Phthalocyanine Green, preferably Phthalocyanine Green CI-74260.

Preferably the total level of pigment in the composition is from 0.01 wt% to 3 wt, more preferably from 0.02 to 2 wt%.

The toothpaste may be a dual phase paste, with the whitening pigments present in one phase.

Compositions according to the invention may comprise oral care enzyme systems such as hydrogen peroxide producing enzyme systems (e.g. the oxidoreductase enzyme glucose oxidase), amyloglucosidase, dextranase and/or mutanase, (optionally in the presence of zinc ion providing compounds and/or 8- hydroxyquinoline derivatives), lactoperoxidase, lactoferrin, lysozyme and mixtures thereof.

Compositions of the invention may comprises fluoride sources such as sodium fluoride, stannous fluoride, sodium monofluorophosphate, zinc ammonium fluoride, tin ammonium fluoride, calcium fluoride, cobalt ammonium fluoride and mixtures thereof. Fluoride ion sources may be added to the compositions at a level of about 0.001 wt. percent to about 10 wt. percent, e.g., from about 0.003 wt. percent to about 5 wt. percent, 0.01 wt. percent to about 1 wt., or about 0.05 wt. percent. In some embodiment, the stannous fluoride is present in an amount of 0.1 wt. percent to 2 wt. percent (0.1 wt percent -0.6 wt. percent) of the total composition weight. Fluoride ion sources may be added to the compositions at a level of about 0.001 wt. percent to about 10 wt. percent, e.g., from about 0.003 wt. percent to about 5 wt. percent, 0.01 wt. percent to about 1 wt., or about 0.05 wt. percent. However, it is to be understood that the weights of fluoride salts to provide the appropriate level of fluoride ion will obviously vary based on the weight of the counter ion in the salt, and one of skill in the art may readily determine such amounts. In some embodiment, the fluoride source is a fluoride salt present in an amount of 0.1 wt. percent to 2 wt. percent (0.1 wt percent -0.6 wt. percent) of the total composition weight (e.g., sodium fluoride (e.g., about 0.32 wt. percent).

Some embodiments of the invention are free from fluoride sources, that is the composition comprises less than 0.01 wt% of a fluoride source.

In one embodiment a preferred class of oral care active for inclusion in the compositions of the invention includes agents for the remineralisation of teeth. The term "remineralisation" in the context of the present invention means the in situ generation of hydroxyapatite on teeth.

A specific example of a suitable agent for the remineralisation of teeth is a mixture of a calcium source and a phosphate source which, when delivered to the teeth results in the in situ generation of hydroxyapatite on teeth.

Illustrative examples of the types of calcium source that may be used in this context (hereinafter termed "remineralising calcium sources") include, for example, calcium phosphate, calcium gluconate, calcium oxide, calcium lactate, calcium glycerophosphate, calcium carbonate, calcium hydroxide, calcium sulphate, calcium carboxymethyl cellulose, calcium alginate, calcium salts of citric acid, calcium silicate and mixtures thereof. Preferably the remineralising calcium source is calcium silicate.

The amount of remineralising calcium source(s) (e.g. calcium silicate) in the composition of the invention typically ranges from 1 to 30%, preferably from 5 to 20% by total weight remineralising calcium source based on the total weight of the oral care composition.

Illustrative examples of the types of phosphate source that may be used in this context (hereinafter termed "remineralising phosphate sources") include, for example, monosodium dihydrogen phosphate, disodium hydrogen phosphate, sodium pyrophosphate, tetrasodium pyrophosphate, sodium tripolyphosphate, sodium hexametaphosphate, potassium dihydrogenphosphate, trisodium phosphate, tripotassium phosphate and mixtures thereof. Preferably the remineralising phosphate source is a mixture of trisodium phosphate and sodium dihydrogen phosphate.

The amount of remineralising phosphate source(s) (e.g. trisodium phosphate and sodium dihydrogen phosphate) in the composition of this invention typically ranges from 2 to 15%, preferably from 4 to 10% by total weight remineralising phosphate source based on the total weight of the oral care composition.

In some embodiments, the oral care compositions comprise an effective amount of one or more antibacterial agents, for example comprising an antibacterial agent selected from halogenated diphenyl ether (e.g. triclosan), triclosan monophosphate, herbal extracts and essential oils (e.g., rosemary extract, tea extract, magnolia extract, thymol, menthol, eucalyptol, geraniol, carvacrol, citral, hinokitol, magnolol, ursolic acid, ursic acid, catechol, methyl salicylate, epigallocatechin gallate, epigallocatechin, gallic acid, miswak extract, sea-buckthom extract), bisguanide antiseptics (e.g., chlorhexidine, alexidine or octenidine), quaternary ammonium compounds (e.g., cetylpyridinium chloride (CPC), CPC-claybenzalkonium chloride, tetradecylpyridinium chloride (TPC), N-tetradecyl-4-ethylpyridinium chloride (TDEPC)), phenolic antiseptics, hexetidine furanones, bacteriocins, ethyllauroyl arginate, arginine bicarbonate, a Camellia extract, a flavonoid, a flavan, halogenated diphenyl ether, creatine, sanguinarine, povidone iodine, delmopinol, salifluor, metal ions (e.g., zinc salts, stannous salts, copper salts, iron salts), propolis and oxygenating agents (e.g., hydrogen peroxide, buffered sodium peroxyborate or peroxycarbonate), phthalic acid and its salts, monoperthalic acid and its salts and esters, ascorbyl stearate, oleoyl sarcosine, alkyl sulphate, dioctyl sulfosuccinate, salicylanilide, domiphen bromide, delmopinol, octapinol and other piperidino derivatives, nisin preparations, chlorite salts; parabens such as methylparaben or propylparaben and mixtures of any of the foregoing. One or more additional antibacterial or preservative agents may optionally be present in the composition in a total amount of from about 0.01 wt. percent to about 0.5 wt. percent, optionally about 0.05 wt. percent to about 0.1 wt. percent or about 0.3 percent. by total weight of the composition.

In some embodiments, the oral care compositions also comprise at least one flavorant, useful for example to enhance taste of the composition. Any orally acceptable natural or synthetic flavorant can be used, including without limitation essential oils and various flavoring aldehydes, esters, alcohols, and similar materials, tea flavors, vanillin, sage, marjoram, parsley oil, spearmint oil, cinnamon oil, oil of wintergreen, peppermint oil, clove oil, bay oil, anise oil, eucalyptus oil, citrus oils, fruit oils, sassafras and essences including those derived from lemon, orange, lime, grapefruit, apricot, banana, grape, apple, strawberry, cherry, pineapple, etc., bean-and nut-derived flavors such as coffee, cocoa, cola, peanut, almond, etc., adsorbed and encapsulated flavorants and the like. Also encompassed within flavorants herein are ingredients that provide fragrance and/or other sensory effect in the mouth, including cooling or warming effects. Such ingredients illustratively include menthol, carvone, menthyl acetate, menthyl lactate, camphor, eucalyptus oil, eucalyptol, anethole, eugenol, cassia, oxanone, a-irisone, , thymol, linalool, benzaldehyde, cinnamaldehyde, N-ethyl-p-menthan-3-carboxamine, N, 2, 3-trimethyl-2- isopropylbutanamide, 3- (1-menthoxy) - propane-1, 2-diol, cinnamaldehyde glycerol acetal (CGA), menthone glycerol acetal (MGA) and the like. One or more flavorants are optionally present in a total amount of from about 0.01 wt. percent to about 5 wt. percent, for example, from about 0.03 wt. percent to about 2.5 wt. percent, optionally about 0.05 wt. percent to about 1.5 wt. percent, further optionally about 0.1 wt. percent to about 0.3 wt. percent and in some embodiments in various embodiments from about 0.01 wt. percent to about 1 wt. percent, from about 0.05 to about 2 percent, from about 0.1 percent to about 2.5 percent, and from about 0.1 to about 0.5 percent by total weight of the composition.

In some embodiments, the oral care compositions comprise at least one sweetener, useful for example to enhance taste of the composition. Sweetening agents among those useful herein include dextrose, polydextrose, sucrose, maltose, dextrin, dried invert sugar, mannose, xylose, ribose, fructose, levulose, galactose, com syrup, partially hydrolyzed starch, hydrogenated starch hydrolysate, ethanol, sorbitol, mannitol, xylitol, maltitol, isomalt, aspartame, neotame, saccharin and salts thereof (e.g. sodium saccharin), sucralose, dipeptide-based intense sweeteners, cyclamates, dihydrochalcones.

One or more sweeteners are optionally present in a total amount depending strongly on the particular sweetener (s) selected, but typically 0.005 wt. percent to 5 wt. percent, by total weight of the composition, optionally 0.005 wt. percent to 0.2 wt. percent, further optionally 0.05 wt. percent to 0.1 wt. percent by total weight of the composition.

In some embodiments, the oral care compositions further comprise an agent that interferes with or prevents bacterial attachment, e.g., ethyl lauroyl arginiate (ELA), solbrol or chitosan, as well as plaque dispersing agents such as enzymes (papain, glucoamylase, etc.).

Mixtures of any of the above described materials may also be used.

The composition according the invention will comprise further ingredients which are common in the art, such as:
antimicrobial agents, e.g. chlorhexidine, sanguinarine extract, metronidazole, quaternary ammonium compounds, such as cetylpyridinium chloride; cetylpyridium chloride clay complex bis-guanides, such as chlorhexidine digluconate, hexetidine, octenidine, alexidine; and halogenated bisphenolic compounds, such as 2,2' methylenebis-(4-chloro-6-bromophenol);
anti-inflammatory agents such as ibuprofen, flurbiprofen, aspirin, indomethacin etc.;
anti-caries agents such as sodium- and stannous fluoride, aminefluorides, sodium monofluorophosphate, sodium trimeta phosphate and casein;
plaque buffers such as urea, calcium lactate, calcium glycerophosphate and strontium polyacrylates;
vitamins such as Vitamins A, C, D, B (preferably B3) and E;
plant extracts;
plant-derivable antioxidants such as flavonoid, catechin, polyphenol, and tannin compounds and mixtures thereof;
desensitising agents, e.g. potassium citrate, potassium chloride, potassium tartrate, potassium bicarbonate, potassium oxalate, potassium nitrate and strontium salts;
anti-calculus agents, e.g. alkali-metal pyrophosphates, hypophosphite-containing polymers, organic phosphonates and phosphocitrates etc.;
biomolecules, e.g. bacteriocins, antibodies, enzymes, etc.;
flavours, e.g. peppermint and spearmint oils;
proteinaceous materials such as collagen;
preservatives;
opacifying agents;
hyaluronic acid;
amino acids such as arginine;
colouring agents;
pH-adjusting agents;
sweetening agents;
mouth feel agents
pharmaceutically acceptable carriers, e.g. starch, sucrose, water or water/alcohol systems etc.; humectants such as glycerol, sorbitol, propyleneglycol, xylitol, lactitol etc.;
polymeric compounds which can enhance the delivery of active ingredients such as antimicrobial agents can also be included;
buffers and salts to buffer the pH and ionic strength of the oral care composition; and
other optional ingredients that may be included are e.g. bleaching agents such as peroxy compounds e.g. potassium peroxydiphosphate, effervescing systems such as sodium bicarbonate/citric acid systems, colour change systems, and so on.

The invention will now be illustrated by the following non-limiting Examples: Examples of the invention are illustrated by a number, comparative Examples by a letter.

### Examples

**Table 1**

| **Ingredient** | **Wt%** | **Wt%** |
|---|---|---|
| | **A** | **1** |
| Sodium carboxymethyl cellulose | 0.7 | 0.7 |
| Sodium lauryl sulphate | 1.6 | |
| Olivoil glutamate BWP* | | 1.6 |
| CAP Betaine 30% | | |
| Hydrated Silica Thickening Med Viscosity | 9.5 | 9.5 |
| Hydrated Silica Medium Abrasivity | 8 | 8 |
| Zinc Citrate Trihydrate | 2 | 2 |
| Sodium Fluoride 98% | 0.32 | 0.32 |
| Sorbitol 70% non-crystallizing grade | 45 | 45 |
| Water and minors | To 100 | To 100 |
| | | |
| Astringency during application | 3.2 | 2.7 |
| Astringency after 5 mins | 2.2 | 1.7 |
| Astrigency adter 10 mins | 1.7 | 1.26 |

| | | |
|---|---|---|
| * Olivoil glutamate BWP* | | |

The products were assessed by a trained sensory panel of 12 panellists and assesses using a scale from 1 to 10, where 10 is high and) low.

The results show that the Example according to the invention was less astringent than the comparative Example.

## Claims

1. A toothpaste composition comprising:
i) a zinc salt and
ii) disodium cocoyl glutamate and sodium olivoyl glutamate.

2. A toothpaste composition according to claim 1 in which ii) disodium cocoyl glutamate and sodium olivoyl glutamate comprise at least 75 wt% of the total surfactant present.

3. A toothpaste composition according to any preceding claim in which the zinc salt comprises zinc citrate.

4. A toothpaste composition according to any preceding claim in which the level of zinc salt is from 0.1 to 5 w5% of the total composition, preferably 1 to 3 wt%

5. A toothpaste composition according to any preceding claim in which the level disodium cocoyl glutamate and sodium olivoyl glutamate is from 0.05 wt% to 4 wt%, preferably 0.5 to 2.5 wt% of the total composition.

6. A toothpaste composition according to any preceding claim which further comprises a silica abrasive.

7. A toothpaste composition according to any preceding claim that further comprises a cellulose gum.

8. A toothpaste composition according to any preceding claim that comprises at least 25 wt% of the total composition of water.
